# EUROPÄISCHE PATENTANMELDUNG

(11) **EP 1 247 566 A1**
(43) Veröffentlichungstag der Anmeldung: **09.10.2002**
(21) Anmeldenummer: 01108275.7
(22) Anmeldetag: 02.04.2001
(51) Int. Cl.: B01J 3/00

(54) **Verfahren zur schnellen thermischen Anregung einer chemischen Reaktion in einer überkritisch fluiden Lösung**

(71) Anmelder: Buback, Michael, Prof. Dr., 37120 Bovenden (DE)
(72) Erfinder: Buback, Michael, Prof. Dr., 37120 Bovenden (DE)
(74) Vertreter: Patentanwälte Rehberg + Hüppe

(57) **Zusammenfassung**

Bei einem Verfahren zur schnellen thermischen Anregung einer chemischen Reaktion in einer Lösung, die sich während der Reaktion in überkritisch fluidem Zustand befindet, wird ein Fluid auf eine Fluidtemperatur aufgeheizt, die oberhalb einer Reaktionstemperatur liegt, wobei die Zusammensetzung des Fluids so abgestimmt ist, daß das Fluid bei der Fluidtemperatur stabil ist und nicht abgebaut wird, dann wird das Fluid mit mindestens einem Reaktionspartner der gewünschten Reaktion vermischt, der sich zuvor auf einer Vorreaktionstemperatur unterhalb der Reaktionstemperatur befindet, wobei die Lösung entsteht und der Reaktionspartner binnen einem Zeitraum auf die Reaktionstemperatur gebracht wird, der kürzer als die Halbwertszeit der gewünschten Reaktion bei der Reaktionstemperatur ist.

## Beschreibung

Die Erfindung bezieht sich auf ein Verfahren zur schnellen thermischen Anregung einer chemischen Reaktion in einer Lösung, die sich während der Reaktion in überkritisch fluidem Zustand befindet, mit den Schritten nach dem Oberbegriff des Anspruchs 1.

Dabei ist der Begriff Reaktionspartner so weit auszulegen, daß er auch eine Substanz, die bei der gewünschten Reaktion nur mit sich selbst umgesetzt oder die bei der Reaktion ab- bzw. umgebaut wird, umfaßt.

Unter einem überkritisch fluiden Zustand ist ein fluider, also nicht fester Zustand zu verstehen, der sich bei reinen Stoffen oberhalb einer kritischen Temperatur T_{c} und oberhalb eines kritischen Drucks p_{c} einstellt. Bei reinen Stoffen endet die Dampfdruckkurve, das ist die Trennungslinie zwischen der Dampfphase und der Flüssigphase im Phasendiagramm, in einem kritischen Punkt der durch die Werte von T_{c}, P_{c} und eine kritische Dichte charakterisiert ist. Bei Stoffgemischen endet die Dampfdruckkurve nicht in einem einzigen Punkt. In überkritisch fluidem Zustand können die Eigenschaften eines Fluids zwischen gasähnlichen und flüssigkeitsähnlichen Zuständen kontinuierlich variiert werden. Eine Übersicht über die Werte von T_{c} und p_{c} verschiedener Substanzen geben P. G. Jessop et al. in dem Beitrag "Supercritical Fluids as Media for Chemical Reactions" in der Monographie "Chemical Synthesis using Supercritical Fluids" Phillip G. Jessop, Walter Leitner (Herausgeber) Wiley-VCH, Weinheim, 1999, in den Tabellen 1.1-1 und 1.1-2.

Es ist bekannt, chemische Reaktionen in überkritisch fluiden Lösungen ablaufen zu lassen. Dabei werden eine hohe Diffusivität und eine damit verbundene niedrige Viskosität sowie oft günstige Lösungseigenschaften in der überkritisch fluiden Phase ausgenutzt, die zudem kontinuierlich durchgestimmt und auf diese Weise speziellen Anforderungen angepaßt werden können. Verschiedene Fluide, auf deren Basis überkritisch fluide Lösungen leicht ausbildbar sind, wie bspw. Kohlendioxid und Wasser, sind im Vergleich zu herkömmlichen flüssigen Lösungsmitteln, die ein vergleichbares Lösungsvermögen bei Normaldruck aufweisen, auch unter Umweltgesichtspunkten von Vorteil. Fluide, die bei Normaldruck und bei normaler Temperatur gasförmig sind, haben bei ihrer Verwendung zur Ausbildung einer überkritisch fluiden Lösung als Reaktionsumgebung den weiteren Vorteil, daß sie sich von gewünschten flüssigen oder festen Reaktionsprodukten durch einfache Entspannung trennen lassen.

Die schnelle thermische Anregung, die bei einem Verfahren nach dem Oberbegriff des Anspruchs 1 schneller als die Halbwertszeit der gewünschten Reaktion sein soll, hat das Ziel, eine von mehreren möglichen chemischen Reaktionen aller vorhandenen Reaktionspartner, die in dem Bereich der relativ hohen Reaktionstemperatur dominant gegenüber den anderen möglichen Reaktionen ist, mit hoher Selektivität, d. h. großer relativer Ausbeute an einem gewünschten Reaktionsprodukt, ablaufen zu lassen.

Aus der US 5 151 533 ist ein Verfahren zur Herstellung von 1,3,5-Estradien(10)-3-ol-17-on (Estron) durch Pyrolyse von 1,4-Androstadien-3,17-dion (ADD) in Gegenwart von 1,2,3,4-Tetrahydronaphthalin (Tetralin) als Wasserstoff-Donator bekannt. Bei diesem Verfahren wird auf eine Fluidtemperatur deutlich oberhalb einer angestrebten Reaktionstemperatur von 450°C-700°C überhitztes Tetralin in einer Mischzone mit einer nur auf eine Vorreaktionstemperatur von bis zu 300°C vorgeheizten Lösung von ADD in Tetralin vermischt, um das ADD sehr schnell in einen höheren Temperaturbereich zu überführen, in dem es mit hoher Selektivität und guter Ausbeute zu Estron umgesetzt werden kann, während es bei niedrigeren Reaktionstemperaturen vorwiegend unerwünschte, nicht zu Estron führende ringöffnende Reaktionen eingeht. Die gewünschte Pyrolysereaktion wird bei einem Druck von 3,5 - 300 MPa und der Reaktionstemperatur von 450°C-700°C durchgeführt, wozu noch eine weitere Aufheizung des Reaktionsgemisches in einem Reaktor vorgesehen ist. Hierbei wird ein überkritisch fluider Zustand des als Lösungsmittel verwendeten Tetralins erreicht. Als nachteilig bei dem bekannten Verfahren stellt sich heraus, daß die Überhitzung des Tetralins auf Temperaturen oberhalb 700°C eine Zersetzung des Tetralins einleitet. Die Halbwertszeit des Tetralinzerfalls bei 700 °C liegt unter einer Sekunde. Es ist daher erforderlich, die Verweilzeit des Tetralins bei Temperaturen oberhalb etwa 675°C deutlich unter einer Sekunde zu halten, um den Tetralin-Verlust in vertretbaren Grenzen zu halten. Temperaturen des überhitzten Tetralins über 700°C sind wegen der Selbstzersetzung des Tetralins mit wirtschaftlich vertretbarem Aufwand nicht zu realisieren. Sie wären für eine sehr schnelle Erhitzung der ADD-Lösung in Tetralin zwecks hoher Estronausbeute aber wünschenswert. Zur Beschleunigung der Anhebung der Lösung aus ADD in Tetralin auf die Reaktionstemperatur kann so nur die relative Menge des überhitzten Tetralins erhöht werden. Besondere Probleme treten auf, wenn es zu Störungen des Tetralindurchflusses kommt. Dann erfolgt auch bei Fluidtemperaturen unter 700°C sehr schnell eine Teer- und Rußbildung in dem Überhitzer für das Tetralin. Ein weiterer Nachteil des bekannten Verfahrens ist, daß sich das beim thermischen Zerfall des 1,4-Androstadien-3,17-dions primär entstehende Methyl-Radikal in der verdichteten überkritisch fluiden Tetralinlösung nur relativ langsam vom zweiten Produkt dieses primären Dissoziationsprozesses entfernt, einer Steroidkomponente mit radikalischer Funktion im Bereich des A-Rings, die als Intermediat auf dem Weg zur Estronbildung anzusehen ist. Es kommt daher in erheblichem Umfang zu einer Käfigreaktion, bei der 4-Methylestron als unerwünschte Verunreinigung des Estrons entsteht.

Der Erfindung liegt die Aufgabe zugrunde, ein Verfahren der eingangs beschriebenen Art aufzuzeigen, bei dem die Nachteile des Stands der Technik beseitigt werden. Insbesondere soll das Verfahren nicht zu Teer- und Rußbildung führen.

Erfindungsgemäß wird diese Aufgabe durch ein Verfahren mit den Merkmalen des Patentanspruchs 1 gelöst. Bevorzugte Ausführungsformen des neuen Verfahrens sind in den Unteransprüchen 2 bis 10 beschrieben.

Das Basismerkmal des neuen Verfahrens ist, daß die Zusammensetzung des Fluids so abgestimmt wird, daß das Fluid bei der Fluidtemperatur chemisch beständig ist. Dies bedeutet, daß das Fluid auch über längere Zeiträume bei der Fluidtemperatur ohne Verlust an Fluid und insbesondere ohne Ruß- und Teerbildung gehalten werden kann. Das Fluid kann in Bezug auf die Reaktionstemperatur auch viel deutlicher überhitzt werden, als dies bislang möglich war. Die Fluidtemperatur kann so hoch gewählt werden, daß der Reaktionspartner allein durch das Vermischen mit dem Fluid auf die Reaktionstemperatur aufgeheizt wird und kein weiteres Aufheizen des Gemisches zum Erreichen der Reaktionstemperatur erforderlich ist. Die Reaktionstemperatur wird quasi-instantan erreicht, wobei die Geschwindigkeit der Temperaturerhöhung des Reaktionspartners nur durch den Vermischungsvorgang mit dem Fluid begrenzt ist. Die bei dieser Reaktionstemperatur mit höchster Selektivität ablaufenden Reaktionen erfolgen damit von Anfang an bevorzugt gegenüber unerwünschten Nebenreaktionen, deren Selektivität bei niedrigeren Temperaturen höher ist.

Als geeignete Fluide zur Verwendung bei dem neuen Verfahren sind neben Edelgasen, deren Einsatz häufig nicht wirtschaftlich ist, Kohlendioxid, Wasser, Ammoniak und Mischungen davon zu nennen. Kohlendioxid ist auch bei sehr hohen Temperaturen, zumindest bis 1300°C, stabil, und es ist im überkritisch fluiden Zustand ein weitestgehend inertes Lösungsmittel für viele organische Substanzen. Auch hat es im überkritisch fluiden Zustand keine korrosiven Wirkungen auf gängige Druckreaktormaterialien. Wasser und Ammoniak können bei erhöhter Temperatur dissoziieren, sind aber in reiner Form stabil. Sie können im überkritisch fluiden Zustand je nach der gewünschten Reaktion nicht nur inertes Lösungsmittel sondern auch Reaktionspartner der Reaktion sein.

Besonders bevorzugt bei dem neuen Verfahren sind aber die Varianten, bei denen das Fluid kein Reaktionspartner der Reaktion ist. Hierbei wiederum ist Kohlendioxid als wirtschaftlich zur Verfügung stehendes Fluid bevorzugt, zumal Kohlendioxid als Abfallprodukt chemischer Prozesse kostengünstig und nahezu unbegrenzt verfügbar ist. Aufgrund der hohen Diffusivität des überkritisch fluiden Kohlendioxids können sich bei einer mehrstufigen Reaktion primär anfallende Zerfallsprodukte rasch voneinander entfernen, so daß die Wahrscheinlichkeit von Käfigreaktionen gering ist.

Das Fluid kann mit einer Reaktionsmischung vermischt werden, die neben dem mindestens einen Reaktionspartner mindestens noch einen weiteren Reaktionspartner oder ein Lösungsmittel enthält.

Die Reaktionslösung kann zusätzlich auch Reaktionshilfsmittel enthalten.

Die Reaktionsmischung, mit der das Fluid vermischt wird, ist vorzugsweise eine flüssige Reaktionslösung, um einerseits eine schnelle vollständige Vermischung mit dem überhitzten Fluid sicherzustellen und um andererseits die Reaktionsmischung bei einem dem überhitzten Fluid entsprechenden hohen Druck bei einer relativ niedriger Vorreaktionstemperatur vorzuhalten. Daneben ist es auch möglich, daß die Reaktionsmischung eine fluide Reaktionslösung ist, die sich ebenso wie das überhitzte Fluid, das sich immer im überkritisch fluiden Zustand befinden wird, schon vor ihrer Vermischung auch in überkritisch fluidem Zustand befindet.

Es ist auch möglich, das Fluid mit mindestens zwei Reaktionspartnern nacheinander zu vermischen. Dabei sollte der Reaktionspartner, der ohne unerwünschte Zersetzung oder andere Nebenreaktionen auf die höhere Temperatur gebracht werden kann, zuerst mit dem Fluid vermischt werden.

In einer besonders bevorzugten Ausführungsform umfassen die Reaktionspartner 1,4-Androstadien-3,17-dion (ADD) und einen Wasserstoffdonator. Bei dem Wasserstoffdonator handelt es sich wiederum vorzugsweise um 1,2,3,4-Tetrahydronaphthalin (Tetralin). Bei dem neuen Verfahren wird das Tetralin aber nicht zur eigentlichen bzw. alleinigen Ausbildung der überkritisch fluiden Lösung verwendet, in der sich die Reaktionspartner befinden, sondern es liegt in einer Mischlösung mit dem Fluid vor. Im Vergleich zu dem bekannten Verfahren zur Herstellung von Estron in überkritisch fluidem Tetralin stellt sich heraus, daß das Kohlendioxid als Fluid alle mit dem Tetralin als Fluid bislang verbundenen Probleme beseitigt. Es kommt nicht zu einer thermischen Selbstzersetzung des Tetralin, weil dieses erst mit seinem gewünschten Reaktionspartner, dem ADD, zusammen auf Temperaturen gebracht wird, bei denen eine Wasserstoffabspaltung erfolgt. Kohlendioxid ist zumindest bis 1300°C chemisch völlig stabil, somit kann durch eine starke Überhitzung des Kohlendioxids auch auf über 850°C, ohne daß hierdurch irgendeine Zersetzung oder gar eine Teer- oder Rußbildung eingeleitet würde, das ADD zusammen mit dem Tetralin in besonders kurzer Zeit auf hohe Reaktionstemperaturen angehoben werden, bei denen Estron mit hoher Ausbeute erhalten wird. Zudem führt die besonders geringe Viskosität von überkritisch fluidem Kohlendioxid, die unter gleichen Druck- und Temperaturbedingungen sowie bei ähnlich reduzierter Dichte um mehr als eine Größenordnung unter derjeningen von Tetralin liegt, zu einem sehr schnellen Abdiffundieren des primär entstehenden Methyl-Radikals, so daß unerwünschte Reaktionsprodukte aus Käfigreaktionen nicht in nennenswertem Umfang anfallen. Weiterhin ist die geringe Viskosität von überkritisch fluidem Kohlendioxid aus strömungsdynamischer Sicht vorteilhaft, da sie kurze Verweilzeiten der überkritisch fluiden Lösung auf der Reaktionstemperatur in einem Reaktor erlaubt und die Ausbildung einer turbulenten Strömung begünstigt. Eine solche turbulente Strömung hat Vorteile nicht nur bei der Vermischung mit Reaktionspartnern, sondern auch bei einer anschließend fortgesetzten Durchmischung der Lösung, in der die gewünschte Reaktion erfolgt. Bei dem neuen Verfahren zur Herstellung von Estron wird durch die Überhitzung nur von Kohlendioxid und durch die hohe Beimischung an inertem Kohlendioxid zu der überkritisch fluiden Lösung auch das Gefährdungspotential aufgrund explosiver Kohlenwasserstoff-Luft-Mischungen herabgesetzt. Am Ende des Verfahrens kann das Kohlendioxid unbedenklich in die Umgebung abgelassen werden. Aufgrund seiner niedrigen Kosten ist eine Rückführung in einem aufwendigen geschlossenen Prozeßkreislauf nicht notwendig.

Ganz allgemein ist es bei dem neuen Verfahren bevorzugt, die Fluidtemperatur um mindestens 200 K, insbesondere sogar um mindestens 500 K, höher einzustellen als die Reaktionstemperatur. Auch der größere Temperaturdifferenzwert von 500 K ist bei Verwendung von Kohlendioxid als Fluid für die meisten Reaktionen problemlos realisierbar. Die Vorreaktionstemperatur liegt vorzugsweise um mindestens 200 K, insbesondere um mindestens 300 K, unterhalb der Reaktionstemperatur. Bevorzugt ist dabei eine geringere Temperaturdifferenz zwischen der Vorreaktions- und der Reaktionstemperatur als zwischen der Reaktions- und der Fluidtemperatur, damit die Reaktionspartner in der Lösung, in der die gewünschte Reaktion erfolgt, nicht zu stark durch das Fluid verdünnt werden müssen, um sie von der Vorreaktionstemperatur auf die Reaktionstemperatur anzuheben. Die aus der Vorreaktionstemperatur und der Fluidtemperatur resultierende Reaktionstemperatur hängt neben diesen Werten selbst von dem Masseverhältnis des Fluids zu den Reaktionspartnern und von den jeweiligen Wärmekapazitäten ab, wobei zusätzlich etwaige Lösungswärmen und bei unterschiedlichen Drücken auch die Temperatur-Druck-Abhängigkeiten eingehen.

Das Masseverhältnis von Kohlendioxid zu ADD/Wasserstoffdonatorlösung bei dem neuen Verfahren zu Herstellung von Estron liegt zweckmäßiger Weise in dem Bereich von 1 bis 10. Als Konzentration des ADD in der ADD/Wasserstoffdonatorlösung sind bei Verwendung von Tetralin als Lösungsmittel und als Wasserstoffdonator Werte zwischen 50 g ADD/kg Lösung und der Sättigungskonzentration des ADD in Tetralin (bei Raumtemperatur) geeignet. Der bevorzugte Konzentrationsbereich liegt zwischen 100 g und 300 g ADD/kg Lösung in Tetralin. Der Temperaturbereich für die gewünschte Reaktionstemperatur liegt im Bereich von 550°C bis 650°C. Er kann in Einzelfällen auch bis 750°C reichen. Generell ist die Reaktionstemperatur vorwiegend nach den Kriterium festzulegen, eine hohe Ausbeute an den gewünschten Reaktionsprodukten zu erhalten. Hieraus ergeben sich für die Herstellung von Estron die voranstehenden konkreten Werte. Damit sich der überkritisch fluide Zustand der Lösung für die Reaktion einstellt, ist aber ganz allgemein darauf zu achten, daß die Reaktionstemperatur zumindest oberhalb der niedrigeren der kritischen Temperaturen der relevanten Fluidphasenkomponenten liegt, wobei es sich hier um die kritische Temperatur des Kohlendioxid von 31°C handelt. Bevorzugt ist es, wenn die Reaktionstemperatur mindestens so hoch ist wie die höchste kritische Temperatur der relevanten Fluidphasenkomponenten. Dies ist hier diejenige des Tetralins von 446°C. Beide Werte werden von den für die Herstellung von Estron bevorzugten Reaktionstemperaturen deutlich überschritten. Der für die Reaktion in der überkritisch fluiden Lösung einzustellende Druck liegt zumindest oberhalb des niedrigeren der kritischen Drücke der relevanten Fluidphasenkomponenten, also hier von Kohlendioxid und Tetralin, so daß der niedrigere kritische Druck derjenige des Tetralins von 3,5 MPa ist. Bevorzugt ist es, wenn der einzustellende Druck mindestens so groß ist wie der höchste kritische Druck der relevanten Fluidphasenkomponenten. Dies ist in der Regel derjenige des Fluids. Hierbei wiederum handelt es sich typisch um den kritischen Druck des Kohlendioxids von 7,4 MPa. Ein als günstig anzusehender Druckbereich liegt zwischen 7,4 und 30 MPa. Es können aber auch höhere Drücke bis 200 MPa angewendet werden.

Alternative Wasserstoffdonatoren zu Tetralin sind in der DE-PS 25 37 254 angeführt.

Um die spätere Vermischung der Reaktionspartner mit dem Fluid zu beschleunigen, kann das Fluid teilweise auch mit den Reaktionspartnern vermischt werden und nicht überhitzt werden. Auf die Möglichkeit, einen Reaktionspartner vor weiteren Reaktionspartnern dem überhitzten Fluid zuzusetzten, wurde bereits oben eingegangen.

Die Erfindung wird jetzt anhand von Ausführungsbeispielen näher erläutert und beschrieben. Dabei skizziert
- Fig. 1: eine Vorrichtung zur Durchführung des Verfahrens, und
- Fig. 2: ist eine Auftragung der Estronausbeute in Prozent über der Reaktionszeit für verschiedene Reaktionstemperaturen.

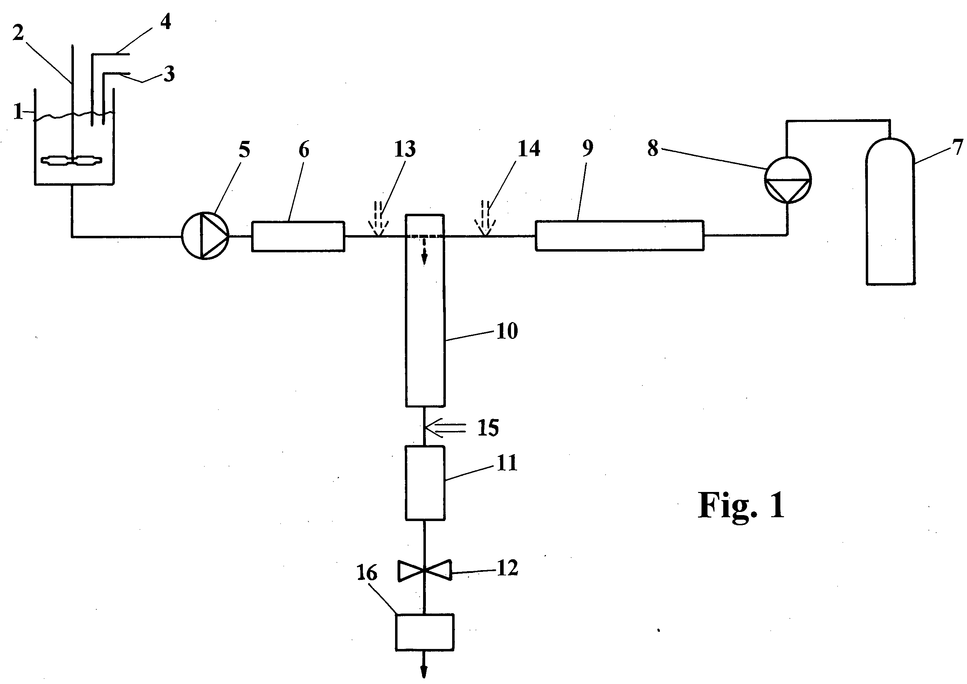

Die in Fig. 1 dargestellte Vorrichtung für eine kontinuierliche Durchführung des neuen Verfahrens weist einen hier links dargestellten Zweig auf, in dem in einem Mischbehälter 1 mit einem Mischwerk 2 eine Mischung aus zwei Reaktionspartnern 3 und 4 hergestellt wird. Bei den Reaktionspartnern 3 und 4 handelt es sich hier um ADD und Tetralin, wobei das ADD in dem Tetralin gelöst wird. Diese Lösung wird in einem Verdichter 5 auf einen Druck von 20 MPa verdichtet, wobei sich die Druckangabe auf eine in einem nachgeschalteten Vorerhitzer 6 erreichbare Vorreaktionstemperatur von 200°C bezieht. In dem hier rechts dargestellten Zweig der Vorrichtung gemäß Fig. 1 wird Kohlendioxid aus einem Druckbehälter 7 in einem Verdichter 8 weiter verdichtet, wobei ein Druck von 20 MPa bezogen auf eine in einem Überhitzer 9 erreichte Temperatur von 860°C bis 1200°C erreicht wird. In einem Reaktor 10 wird aus den beiden Stoffströmen der Lösung von ADD in Tetralin von links und dem Kohlendioxid von rechts durch Vermischen eine Lösung hergestellt, die aufgrund der Wärmeenergie des überhitzten Kohlendioxids eine Reaktionstemperatur im Bereich von 550°C bis 650°C aufweist und deren Zustand überkritisch fluid ist, wobei die überkritisch fluide Phase im wesentlichen von dem Kohlendioxid aber anteilig auch von dem Tetralin ausgebildet wird. In der überkritisch fluiden Lösung erfolgt über einen kurzen Zeitraum von 0,05 bis 2 Sekunden in der Anwesenheit des Tetralins eine Pyrolyse des ADD zu Estron. Die Reaktion wird durch Abkühlung in einer ungeheizten und nicht isolierten, aus dem Reaktor 10 führenden Kapillare und durch eine Expansion der überkritisch fluiden Phase in eine Expansionskammer 11 und dabei erfolgendes Abfallen der Temperatur auf unter 400°C beendet. Der Massestrom wird über ein Ventil 12 geregelt. Der Gasstrom wird zur Abscheidung von flüssigen und festen Komponenten bei Normaldruck durch einen auf ≤ 0°C gehaltenen Behälter 16 geführt. Die bis hierher beschriebene Reaktion kann dadurch modifiziert werden, daß gemäß einem Pfeil 13 auch dem von links kommenden Stoffstrom Kohlendioxid zugesetzt wird oder gemäß einem Pfeil 14 dem von rechts kommenden Stoffstrom Tetralin. Im letzteren Fall ist aber darauf zu achten, daß das Tetralin nur sehr kurze Zeit den hohen Temperaturen des überhitzten Kohlendioxids ausgesetzt wird. Anderenfalls kommt es zu einer Zersetzung des Tetralins unter Wasserstoffabspaltung. Darüber hinaus besteht die Möglichkeit, gemäß einem Pfeil 15 der Lösung nach dem Reaktor ein Lösungsmittel zuzusetzen, um die Abkühlung weiter zu beschleunigen und um Verstopfungen der Vorrichtung zu vermeiden.

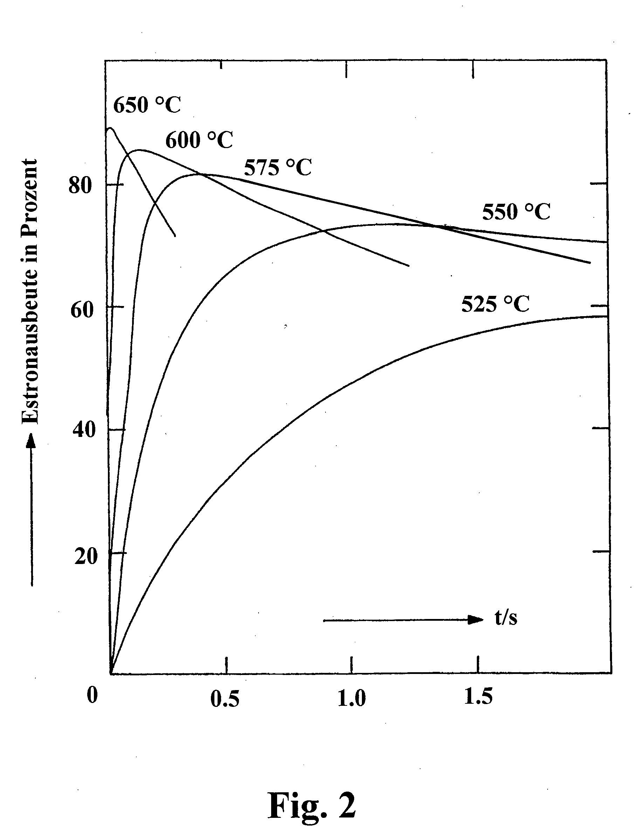

Fig. 2 läßt erkennen, daß die Ausbeuten bei der Umsetzung von ADD zu Estron dadurch verbessert werden können, daß die Reaktionstemperatur erhöht und die Reaktionsdauer verkürzt wird. Für beide Maßnahmen schafft die vorliegende Erfindung die notwendige Basis. Durch die Verwendung von Kohlendioxid als Wärmeträger kann ohne weiteres die gesamte Wärmezufuhr für die Reaktionspartner nur durch das CO₂ bewirkt werden und damit in sehr kurzer Zeit. Es kann auch eine sehr hohe Reaktionstemperatur erreicht werden, ohne daß einer der Reaktionspartner im Vorfeld thermisch beansprucht wird. Unterhalb einer Temperatur von 300°C wird das Tetralin weder alleine noch in Verbindung mit dem ADD binnen üblicher kurzer Verweilzeiten in dem Vorerhitzer oder den nachgeschalteten Leitungen in nennenswertem Umfang umgesetzt.

Als weiteres Beispiel für eine thermisch schnell aktivierte Reaktion im Sinne der vorliegenden Erfindung kann auf das schnelle Überführen einer wässrigen, Salz enthaltenden Lösung in den überkritisch fluiden Zustand verwiesen werden. Es ist bekannt, daß wässrige Salzlösungen kurz vor Erreichen der überkritisch fluiden Phase äußerst korrosive Eigenschaften entwickeln, die ihre Handhabung sehr erschweren. Durch das schnelle Erreichen einer überkritisch fluiden Phase bei dem Verfahren der Erfindung, indem stark überhitztes Wasser zum Aufheizen der wässrigen Salzlösung verwendet wird, kann der hochkorrosive Zustand der Salzlösungen vermieden werden und es können andere gewünschte thermische Reaktionen in der wässrigen Lösungen angeregt und mit kurzer Verweilzeit in einem Reaktor durchgeführt werden.

### BEZUGSZEICHENLISTE

- 1 -: Mischbehälter
- 2 -: Mischwerk
- 3 -: Reaktionspartner
- 4 -: Reaktionspartner
- 5 -: Verdichter
- 6 -: Vorerhitzer
- 7 -: Druckbehälter
- 8 -: Verdichter
- 9 -: Überhitzer
- 10 -: Reaktor

- 11 -: Expansionskammer
- 12 -: Ventil
- 13 -: Pfeil
- 14 -: Pfeil
- 15 -: Pfeil
- 16 -: Behälter

## Patentansprüche

1. Verfahren zur schnellen thermischen Anregung einer chemischen Reaktion in einer Lösung, die sich während der Reaktion in überkritisch fluidem Zustand befindet, mit den Schritten:
- Aufheizen eines Fluids auf eine Fluidtemperatur, die oberhalb einer Reaktionstemperatur liegt,
- Vermischen des Fluids mit mindestens einem Reaktionspartner der gewünschten Reaktion, der sich zuvor auf einer Vorreaktionstemperatur unterhalb der Reaktionstemperatur befindet,
- wobei die Lösung entsteht und der Reaktionspartner binnen einem Zeitraum auf die Reaktionstemperatur gebracht wird, der kürzer als die Halbwertszeit der gewünschten Reaktion bei der Reaktionstemperatur ist,
**dadurch gekennzeichnet, daß** die Zusammensetzung des Fluids so abgestimmt wird, daß das Fluid bei der Fluidtemperatur stabil ist und nicht abgebaut wird.

2. Verfahren nach Anspruch 1, **dadurch gekennzeichnet, daß** das Fluid aus der Gruppe ausgewählt wird, die aus Kohlendioxid, Wasser, Ammoniak und Mischungen davon besteht.

3. Verfahren nach Anspruch 1 oder 2, **dadurch gekennzeichnet, daß** das Fluid ein Reaktionspartner der Reaktion ist.

4. Verfahren nach Anspruch 1 oder 2, **dadurch gekennzeichnet, daß** das Fluid kein Reaktionspartner der Reaktion ist.

5. Verfahren nach Anspruch 4, **dadurch gekennzeichnet, daß** das Fluid Kohlendioxid ist.

6. Verfahren nach Anspruch 4 oder 5, **dadurch gekennzeichnet, daß** das Fluid mit einer Reaktionsmischung vermischt wird, die neben dem mindestens einen Reaktionspartner mindestens noch einen weiteren Reaktionspartner oder ein weiteres Lösungsmittel enthält.

7. Verfahren nach Anspruch 6, **dadurch gekennzeichnet, daß** die Reaktionsmischung eine flüssige Reaktionslösung ist.

8. Verfahren nach einem der Ansprüche 1 bis 7, **dadurch gekennzeichnet, daß** das Fluid mit mindestens zwei Reaktionspartner nacheinander vermischt wird.

9. Verfahren nach einem der Ansprüche 1 bis 8, **dadurch gekennzeichnet, daß** die Reaktionspartner 1,4-Androstadien-3,17-dion (ADD) und einen Wasserstoffdonator umfassen.

10. Verfahren nach Anspruch 9, **dadurch gekennzeichnet, daß** die Fluidtemperatur größer als 850° C ist.
